Europäisches Patentamt

European Patent Office

Office européen des brevets

(11) Veröffentlichungsnummer: **0 188 008**
**A2**

## (12) EUROPÄISCHE PATENTANMELDUNG

(21) Anmeldenummer: 85116671.0

(22) Anmeldetag: 31.12.85

(51) Int. Cl.⁴: **G 01 N 33/556**
**//G01N33/49**

(30) Priorität: 18.01.85 DE 3501496

(43) Veröffentlichungstag der Anmeldung:
23.07.86 Patentblatt 86/30

(84) Benannte Vertragsstaaten:
AT BE CH DE FR GB IT LI LU NL SE

(71) Anmelder: BEHRINGWERKE Aktiengesellschaft
Postfach 1140
D-3550 Marburg 1(DE)

(72) Erfinder: Kolde, Hans-Jürgen, Dr.
Höhenweg 54
D-3550 Marburg 1(DE)

(74) Vertreter: Meyer-Dulheuer, Karl-Hermann, Dr. et al,
HOECHST Aktiengesellschaft Zentrale Patentabteilung
Postfach 80 03 20
D-6230 Frankfurt/Main 80(DE)

(54) Verfahren zur Bestimmung der Aktivität des Komplementsystems des Blutes.

(57) Es wird ein Verfahren beschrieben zur Bestimmung der Aktivität des Komplementsystems von humanem oder säugetierischem Blut mittels photometrischer Verfolgung der Lyse von sensitivierten Erythrozyten, worin als Probenmaterial Blutplasma verwendet wird.

EP 0 188 008 A2

0188008

BEHRINGWERKE AKTIENGESELLSCHAFT    85/B 002
Dr. Ha/Sd.

Verfahren zur Bestimmung der Aktivität des Komplementsystems des Blutes

---

Die Erfindung betrifft ein Verfahren zur Bestimmung der
Aktivität des Komplementsystems des Blutes von Menschen
oder Säugetieren.

Das Komplementsystem übt im humanen und tierischen Organismus eine ganze Reihe von Funktionen aus. Als wichtigste gelten die Chemotaxis, Opsonierung und Lyse von
Zellen. Es unterstützt das Immunsystem des Körpers, das
aus Antikörpern und immunkompetenten Zellen besteht.
Eine ganze Reihe von Berührungspunkten besteht zwischen
Komplement- und anderen Regulationssystemen im Blut,
beispielsweise dem Gerinnungs- und Fibrinolysesystem. So
kann eine durch Komplement vermittelte Lyse von Leukozyten Thromboplastin freisetzen, was zur Aktivierung des
exogenen Gerinnungssytems führt. Andererseits können
aber auch aktivierte Gerinnungsfaktoren wie Thrombin
Proteine des Komplementsystems aktivieren. Auch kommt
dem wichtigsten Gerinnungsinhibitor, Antithrombin III,
eine regulatorische Funktion im Komplementsystem zu. Die
bei der Aktivierung des Gerinnungssystems gleichzeitig
induzierte Bildung von Plasmin führt auch zu einer Aktivierung der Komplementfaktoren Clr und Cls, bei längerer
Einwirkung aber zu deren Desaktivierung. Der bei der
Gerinnung aktivierte Faktor XIIa verbraucht den wichtigen Komplement-Inhibitor Cl-Inaktivator und aktiviert
ebenfalls Komplement.

Ein bekanntes Verfahren zur Bestimmung des Komplements ist die $CH_{50}$-Bestimmung nach Mayer, die üblicherweise in Serum durchgeführt wird. In Serum sind jedoch Gerinnungs- und Fibrinolysefaktoren bereits aktiviert; auch die Blutplättchen haben ihren Inhalt abgegeben und Inhibitoren wie das AT III sind teilweise verbraucht, so daß die volle biologische Wirkung des Komplementsystems gestört ist.

Es ist bekannt, daß in gewissen Fällen bei Verwendung von Serum pathologische Werte bei $CH_{50}$-Bestimmungen erhalten werden, obwohl die Bestimmung einzelner Komplementfaktoren und der $CH_{50}$-Aktivität in Plasma normale Werte liefern (Übersichten: Th.F. Lint, Laboratory Detection of Complement Activation and Complement Deficiencies, Am.J.Med.Technol. (1982) 48, 743 und A.T. Luskin und M.C. Tokin, Alterations of Complement Component in Disease, Am.J.Med.Technol. (1982) 48, 749). Aus diesen durch Einwirkung des Gerinnungssystems in vitro entstandenen Komplementdefekten erscheint eine Bestimmung in Plasma vorteilhafter zu sein als in Serum.

Ein weiteres Verfahren zur Erfassung der Komplementaktivität in Serum ist in Z. Naturforschung 20b, 569-574 (1965) beschrieben.

In dieser Methode wird die Lyse einer Schaferythrozytensuspension, die mit Kaninchenantikörpern gegen Schaferythrozyten sensibilisiert wurden, durch Serum erfaßt und die Zeit bestimmt, in der die Extinktion der Suspension auf die Hälfte gesunken ist. Die Messung der Zeit liefert damit eine Aussage über die Aktivität des Serums. Dieses Verfahren ist jedoch umständlich und führt insbesondere bei geringen Komplementaktivitäten zu verhältnismäßig langen Meßzeiten.

Überraschenderweise wurde gefunden, daß die Aktivität des Komplementsystems von Blut bestimmt werden kann, indem Citrat-Plasma mit in einem Calcium- und Magnesiumionen enthaltenden Puffer suspendierten und mit Antikörpern sensitivierten Erythrozyten versetzt, die Zeit bis zum Erreichen einer bestimmten Extinktionserniedrigung gemessen und mit der Zeit in Beziehung gesetzt wird, die an einem Plasma-Standard gemessen wird.

Die Extinktionserniedrigung braucht lediglich eine Differenz von zum Beispiel 0,1 der optischen Dichte (O.D.) der Suspension direkt nach Vereinigen von Probe und Reagenz zu betragen, so daß sich eine vorteilhafte Verkürzung des Zeitbedarfs für eine Bestimmung besonders bei niederen Komplementaktivitäten und Vereinfachung verglichen mit dem bekannten optischen Verfahren für Serum ergibt.

Gegenstand der Erfindung ist daher ein kinetisches Verfahren zur Bestimmung der Aktivität des klassichen Wegs des Komplementsystems von humanem oder säugetierischem Blut mittels photometrischer Verfolgung der Lyse von mit Antikörpern sensitivierten Schaferythrozyten in einem Calcium- und Magnesiumionen enthaltenden Puffer, dadurch gekennzeichnet, daß ein Citratplasma des Blutes als Probematerial verwendet wird.

Eine besonders vorteilhafte neue erfindungsgemäße Ausführungsform besteht darin, daß die Zeit gemessen wird, in der die optische Dichte des Systems bei 578 nm um 0,025 bis 0,2, vorzugsweise 0,05 bis 0,15, abnimmt. Alternativ können auch andere Wellenlängen von 540 bis zu 800 nm verwendet werden, bei denen man die Trübung einer Erythrozytensuspension messen kann.

Es ist vorteilhaft, die Komplementaktivität in einem Probenmaterial zu bestimmen, in dem das Gerinnungssystem weitgehend intakt ist wie in Citratplasma. Der Test kann in dieser Form jedoch auch - mit den erwähnten Einschränkungen - an Serum durchgeführt werden.

Bei der Gewinnung von Plasma für eine Untersuchung der Komplementfunktion muß sichergestellt sein, daß das Komplementsystem durch ein Antikoagulans, das den Entzug der komplement- und gerinnungsaktiven Calciumionen und auch der für das Komplementsystem erforderlichen Magnesiumionen bewirkt, nicht inaktiviert wird.

In dem hier beschriebenen Verfahren kann das für Gerinnungsuntersuchungen übliches Citratplasma eingesetzt werden, nicht jedoch EDTA-Plasma, das offensichtlich zu einer Inaktivierung des klassischen Wegs des Komplementsystems führt. Im Gegensatz dazu wurde in der oben zitierten Literatur auch auf die Verwendung von EDTA-Plasma hingewiesen (Lint und Luskin und Tokin). Auch Heparinplasma kann im fotometrischen Test eingesetzt werden. Es wird fotometrisch im langwelligen Bereich des sichtbaren Lichtes die Turbiditätsänderung der durch Komplement vermittelten Lyse von suspendierten, mit Antikörpern gegen Schaferythrozyten vom Kaninchen sensibilisierten Schaferythrozyten gemessen. Die Erythrozyten sind in einem calcium- und magnesium-ionenhaltigen Puffer suspendiert. Alternativ kann eine gereinigte Immunglobulinfraktion oder auch ein Antiserum zur Sensibilisierung eingesetzt werden.

Da die Reaktionsgeschwindigkeit mit zunehmender Temperatur ansteigt, ist das Arbeiten bei erhöhter Temperatur und die Verwendung eines vortemperierten Reagenzes vorteilhaft. Es wird vorzugsweise bei 37°C gearbeitet. Die in der Z. Naturforschung beschriebene Methode hingegen

benutzt auf Eis gelagertes Reagenz, wobei eine Temperaturkonstanz während der Bestimmung nicht gewährleistet ist, da es während der Reaktion zu einer stetigen Erwärmung des Reagenz kommt, so daß aktive Seren in kurzer Zeit bei relativ geringer Temperatur reagieren, weniger aktive jedoch bei höherer Temperatur. Überraschenderweise ist jedoch das in Beispiel 1 erläuterte Reagenz auch bei 37°C über mindestens 8 Stunden stabil und kann daher bei dieser Temperatur gut eingesetzt werden.

Der zur Suspendierung der sensibilisierten Schaferythrozyten verwendete Puffer hat vorteilhafterweise einen pH-Wert von 6 bis 8, vorzugsweise von 7 bis 7,5. Als Puffersubstanzen können beispielsweise Veronal , HEPES, TRIS oder Imidazol, bevorzugt HEPES eingesetzt werden. Geeignete Konzentrationen der Puffersubstanz sind 5-100, vorzugsweise 20 mM.

Die Reaktionsgeschwindigkeit hängt auch von der Beladungsdichte der Schaferythrozyten ab. Daher ist eine optimale Dosierung des Kaninchenantikörpers gegen Schaferythrozyten wichtig. Eine geeignete Konzentration ist in Beispiel 1 angegeben. Die Zelldichte selbst spielt jedoch für die Reaktionszeiten nur eine untergeordnete Rolle, wenn man die erfindungsgemäße Methode benutzt. Dagegen ist die Zeit für die 50 %-Hämolyse bei höherer Zelldichte von der Erythrozytenkonzentration abhängig.

Das folgende Beispiel erläutert die Erfindung.

Beispiel 1

Herstellung eines erfindungsgemäßen Reagenz

200 μl einer Suspension von gewaschenen Schaferythrozyten wurden mit 200 μl einer Lösung von Kaninchenanti-

körpern gegen Schaferythrozyten (Ambozeptor 6000,
(Behringwerke AG, Marburg, Bundesrepublik Deutschland;
Verdünnung 1:50, oder auch eine gereinigte Immunglobulinfraktion dieses Antiserums) gemischt und mit einem
Puffer, der 0,15 mmol/l $CaCl_2$, 0,5 mmol/l $MgCl_2$, 20
mmol/l Hepes, pH 7,3, 150 mmol/l NaCl und 1 g/l Gelatine
enthielt, auf 10 ml aufgefüllt. Die Zelldichte der Erythrozytensuspension betrug $2 \times 10^7$ Zellen/ml. Nach etwa
10 min ist das Reagenz gebrauchsfertig. Vor Verwendung
im fotometrischen Test wurde die Suspension of 37°C
erwärmt und eventuell sedimentierte Erythrozyten vorsichtig aufgeschüttelt.
Während einer Bestimmung kann jedoch die Sedimentation
der Zellen vernachlässigt werden.


Beispiel 2


Meßverfahren

50 µl Human-Citratplasma wurden in eine auf 37°C vorgewärmte Küvette pipettiert und 500 µl des voranstehend
hergestellten Reagenzes, das auf 37°C gehalten worden
war, zugesetzt. Nach Vermischung der Komponenten wurde
die Extinktion bei 578 nm bestimmt. Als Maßparameter
wird die Zeit für das Erreichen einer Extinktionsdifferenz von -0,1 O.D., die durch die Lysis von Erythrozyten durch Komplement hervorgerufen wird, bestimmt.
Diese Zeit ist selbstverständlich kürzer als die für
eine Lysis der Hälfte der Erythrozyten. Insbesondere
bei pathologischen Plasmen mit verringerter Komplementaktivität zeigt sich der Zeitgewinn bei Verwendung des
erfindungsgemäßen Verfahren im Vergleich zu einer Bestimmung der für die halbmaximalen Lyse nötigen Zeit
(Abbildung 1).

## Beispiel 3

__Empfindlichkeit des Verfahrens für Komplementdefekte__
1 ml Citratplasma wurde mit 200 µl einer Lösung von
Fab´-Fragmenten von Kaninchenantikörpern (Behringwerke
AG, Marburg, Bundesrepublik Deutschland) gegen die Komplementfaktoren Clq, Cls, C3, C4 oder C5 des klassichen
Weges oder Faktor B des alternativen Weges versetzt. Zur
Verdünnung der Lösung der Fab´-Fragmente wurde sie mit
physiologischer Kochsalzlösung versetzt.

## Tabelle 1

Einfluß von Antikörperfragment Fab´ gegen Clq, Cls, C3,
C4, C5 und Faktor B auf die Reaktionszeit

Ansatz:     60 µl Plasma
            30 µl Fab´- bzw. NaCl-Lösung
           600 µl Suspension sensitivierter Erythrozyten
                  (37°C)

| Fab´-Konzentra-tion (% des Aus-gangswertes) | Clq | Cls | C3 | C4 | C5 | B |
|---|---|---|---|---|---|---|
| 0 | 0,84 | 0,85 | 0,84 | 0,83 | 0,85 | 0,83 |
| 3,3 | 1,9 | 0,87 | 0,83 | 0,96 | 1,47 | 0,84 |
| 8,25 | 3,7 | 0,9 | 0,87 | 1,66 | 11,9 | 0,82 |
| 16,5 | > 16 | 1,4 | 0,9 | > 16 | > 16 | 0,85 |
| 50 | > 16 | 10 | 1,68 | > 16 | > 16 | 0,84 |
| 100 | > 16 | > 16 | > 16 | > 16 | > 16 | 0,83 |

Zeit für Delta E = -0,1 in min bei Zusatz von Fab´ gegen

In der Tabelle 1 sieht man, wie steigende Fab´-Konzentrationen gegen Clq, Cls, C3, C4 und C5 die Reaktionszeit gegenüber der Kontrolle (Fab´-Konzentration = 0; nur NaCl-Lösung) verlängern. Der Antikörper gegen Faktor B zeigt dagegen keinen Einfluß.

Dieses Ergebnis zeigt, daß das Reagenz alle untersuchten Faktoren des klassischen Weges empfindlich anzeigt, nicht jedoch einen Mangel an Faktor B aus dem alternativen Weg des Komplements.

Beispiel 4

Bestimmung eines Einzelfaktors (C4)
C4-Mangelserum wurde von Meerschweinchen mit genetisch bedingtem C4-Mangel erhalten. Zur Bestimmung der Aktivität des Faktors C4 wurde humanes Citratplasma mit NaCl ausverdünnt.
20 µl Plasma wurde mit einem Überstand von 100 µl Mangelserum versetzt und durch Zusatz sensitivierter Erythrozyten die Reaktion gestartet.Bei doppelt logarithmischer Auftragung wurde eine lineare Beziehung zwischen C4-Konzentrationen und Reaktionszeit gefunden.
Ein vergleichbarer Ansatz läßt sich mit einem C2-Mangelserum für C2 durchführen.

Beispiel 5

Einfluß von Proteasen des Gerinnungssystems auf die Komplementaktivität
Den Einfluß von Thrombin, Kallikrein und Plasmin, die während der Gerinnung aktiviert werden, auf die Komplementaktivität von Plasma zeigt Tabelle 2. Aus diesen Ergebnissen läßt sich ableiten, daß das Gerinnungssystem die Komplementaktivität beeinflussen kann. So werden durch Thrombin- und Kallikreineinwirkung auf Plasma längere Meßzeiten erhalten.

Ansatz:

20 µl Enzym-Lösung und 60 µl Citratplasma wurden 60 sec bei 37°C inkubiert. Dann wurden 600 µl Reagenz nach Beispiel 1 zugegeben. Es wurde die Zeit für Delta-E = 0,1 bestimmt.

| Thrombin (IU/l) | Zeit (sec) | Kallikrein (BAEE) | Zeit (sec) |
|---|---|---|---|
| 0 | 45 | 0 | 45 |
| 1 | 45,6 | 5,75 | 46 |
| 4 | 48 | 11,5 | 57 |
| 6 | 62,4 | 23 | 75 |

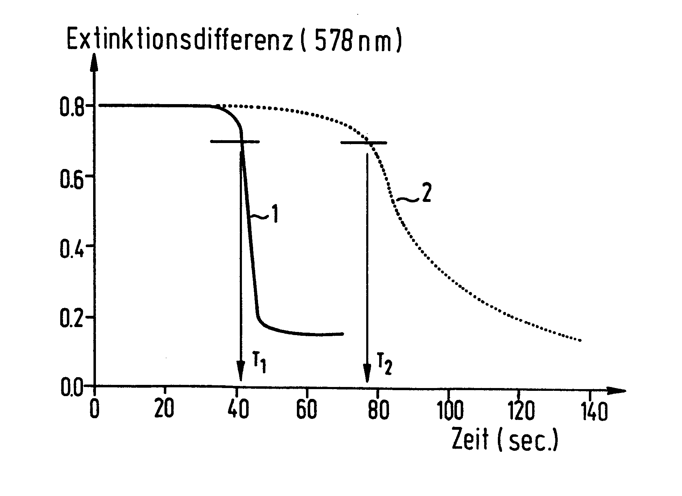

Patentansprüche:

1. Verfahren zur Bestimmung der Aktivität des Komplementsystems von humanem oder säugetierischem Blut mittels photometrischer Verfolgung der Lyse von sensitivierten Erythrozyten in einem Calcium- und Magnesiumionen enthaltenden Puffer, dadurch gekennzeichnet, daß als Probenmaterial Blutplasma, das als Antikoagulans Zitronensäure oder ein Salz davon enthält, verwendet wird.

2. Verfahren nach Anspruch 1, dadurch gekennzeichnet, daß die Zeitdauer gemessen wird, in der die optische Dichte des Systems bei einer Wellenlänge von 540 bis 800 nm, vorzugsweise 578 nm, um 0,025 bis 0,2, vorzugsweise 0,05 bis 0,15, abnimmt.

3. Verfahren nach Anspruch 1, dadurch gekennzeichnet, daß die Erythrozyten solche vom Schaf sind.

4. Verfahren nach Anspruch 1, dadurch gekennzeichnet, daß die Erythrozyten mit Antikörpern sensitiviert wurden, die durch Immunisieren von Kaninchen mittels Erythrozyten-Antigenen vom Schaf erhalten wurden.

5. Verfahren nach Anspruch 1, dadurch gekennzeichnet, daß der Puffer ein HEPES enthaltender Puffer, Konzentrationsbereich 5-100, vorzugsweise 20 mM, pH 6,5-8,5, vorzugsweise 7,3, ist.

6. Verfahren nach Anspruch 1, dadurch gekennzeichnet, daß der Puffer Calciumionen in einer Konzentration von 0,05-0,5, vorzugsweise 0,15 mmol/l, und Magnesiumionen in einem Konzentrationsbereich von 0,1-2 mmol/l, vorzugsweise 0,5 mmol/l, sowie Kochsalz zum Erreichen einer physiologischen Leitfähigkeit (15 mS ± 2,5 mS) enthält.

7. Verfahren nach Anspruch 1, dadurch gekennzeichnet, daß ein Mangelplasma oder Mangelserum eines Faktors des klassischen Wegs des Komplements im Überschuß zugesetzt und die Aktivität dieses Mangelfaktors im Probenmaterial bestimmt wird.